# EUROPEAN PATENT APPLICATION

(11) **EP 4 696 777 A1**
(43) Date of publication of application: **18.02.2026**
(21) Application number: 24778182.6
(22) Date of filing: 28.03.2024
(51) Int. Cl.: C12N 9/22, C07K 19/00, C12N 15/113, C12N 15/85, C12N 5/10, C12Q 1/68

(54) **CAS13 PROTEIN, AND CRISPR-CAS SYSTEM AND USE THEREOF**

(30) Priority: 28.03.2023 CN 202310318479
(71) Applicant: Yoltech Therapeutics Co., Ltd, Shanghai 201109 (CN)
(72) Inventor: WU, Yuxuan, Shanghai 201109 (CN)
(74) Representative: CH Kilger Anwaltspartnerschaft mbB
(86) International application number: PCT/CN2024/084584
(87) International publication number: WO 2024/199396

(57) **Abstract**

A Cas13 protein, a CRISPR-Cas system, and use thereof. The amino acid sequences of the Cas13 protein are as shown in SEQ ID NO. 1 to SEQ ID NO. 9, and are respectively named as Cas13a_10, Cas13a_11, Cas13b_3, Cas13b_4, Cas13b_5, Cas13T_9, Cas13T_15, Cas13T_16, and Cas13Z_2; these nine Cas13 proteins have low homology with the previously reported Cas13 proteins, and exhibit a RNA nuclease activity, showing great prospects in gene editing

## Description

### TECHNICAL FIELD

The present disclosure relates to a Cas13 protein, a CRISPR-Cas system, and use thereof, and pertains to the field of biotechnology.

### BACKGROUND

The CRISPR (Clustered Regularly Interspaced Short Palindromic Repeats) system is developed by bacteria and archaea for defending against invading phage DNA. The CRISPR system is categorized into two families: Class 1 systems are further divided into types I, III, and IV, while Class 2 systems are divided into types II, V, and VI. These 6 system types are further subdivided into 19 subtypes. Many prokaryotes contain multiple CRISPR-Cas systems, which indicates that these systems are compatible and may share components.

The CRISPR/Cas13 system is a CRISPR system capable of targeting and cleaving RNA. This system comprises a Cas13 protein and a CRISPR RNA (guide RNA), which assemble to form a guide RNA-directed RNA-targeting effector complex. Currently, four subtypes have been identified in the Cas13 family, including Cas13a, Cas13b, Cas13c, Cas13d, and the like.

Compared with RNAi and CRISPRi technologies, the CRISPR/Cas13 system can achieve higher RNA degradation while having a lower off-target probability. However, the Cas13 proteins identified so far still have drawbacks such as large molecular weight and low cleavage activity, which impair the editing performance of the CRISPR/Cas13 system on target RNA. CRISPR/Cas13 systems derived from different microbial sources exhibit diversity in function and structure. Therefore, there remains a need to continue exploring more Cas13 proteins to construct CRISPR-Cas13 systems with superior performance.

### SUMMARY

The present disclosure provides a novel Cas13 protein and a CRISPR-Cas13 system to address the aforementioned problems.

In one aspect of the present disclosure, provided is a Cas13 protein, wherein the Cas13 protein is a protein as defined in (a) or (b) below:
(a) a protein comprising an amino acid sequence represented by SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, or SEQ ID NO. 9;
(b) a protein being derived by substituting, deleting, or adding one or more amino acids in the amino acid sequence defined in (a), and substantially retaining the biological function of the sequence from which it is derived.

In some preferred embodiments, the protein in (b) has an amino acid sequence having at least 85% sequence identity to that in (a), and substantially retains the biological function of the sequence from which it is derived.

In some preferred embodiments, the protein in (b) has an amino acid sequence having at least 95% sequence identity to that in (a), and substantially retains the biological function of the sequence from which it is derived.

In a second aspect of the present disclosure, provided is a fusion protein, wherein the fusion protein comprises the aforementioned Cas13 protein and a heterologous functional domain.

In some preferred embodiments, the heterologous functional domain comprises at least one selected from the group consisting of a deaminase, a reporter protein, a detection label, a localization signal, a protein targeting domain, a DNA-binding domain, an epitope tag, a transcriptional activation domain, a transcriptional repression domain, a nuclease, a methylation-active enzyme, a demethylase, a transcript release factor, an RNA-cleaving polypeptide, a nucleic acid ligase, a histone modification activator, and a nucleic acid-binding activator.

In some preferred embodiments, the heterologous functional domain is fused to the N-terminus or C-terminus of the Cas13 protein, or the heterologous functional domain is fused internally within the Cas13 protein.

In some preferred embodiments, the localization signal is a nuclear localization signal (NLS) or a nuclear export signal (NES).

In a third aspect of the present disclosure, provided is a CRISPR-Cas system, wherein the CRISPR-Cas system comprises the Cas13 protein of the first aspect and a guide RNA, or the CRISPR-Cas system comprises the fusion protein of the second aspect and a guide RNA; and the guide RNA is capable of binding to the Cas13 protein to form a complex and guiding the complex to contact a target RNA.

In some preferred embodiments, the guide RNA comprises a spacer sequence and a direct repeat sequence; the spacer sequence is between 15-60 nucleotides; the direct repeat sequence is between 15-40 nucleotides.

In some preferred embodiments, the spacer sequence is between 25-35 nucleotides.

In some preferred embodiments, the spacer sequence is about 30 nucleotides.

In some preferred embodiments, there is complementarity of at least 15 nucleotides between the spacer sequence and the target RNA.

In a fourth aspect of the present disclosure, provided is a polynucleotide, wherein the polynucleotide encodes the Cas13 protein of the first aspect, or the polynucleotide encodes the fusion protein of the second aspect, or the polynucleotide encodes the CRISPR-Cas system of the third aspect.

In a fifth aspect of the present disclosure, provided is a vector, wherein the vector carries the polynucleotide of the fourth aspect.

In some preferred embodiments, the polynucleotide carried by the vector is linked to regulatory elements.

In some preferred embodiments, the regulatory elements comprise at least one selected from the group consisting of a promoter, an enhancer, a polyadenylation signal, a terminator, a protein degradation signal, a transposon, a leader sequence, a polyadenylic acid sequence, and a marker gene.

In some preferred embodiments, the vector comprises at least one selected from the group consisting of a viral vector and a non-viral vector.

In some preferred embodiments, the viral vector comprises at least one selected from the group consisting of a retroviral vector, a phage vector, an adenoviral vector, an adeno-associated viral vector, a vaccinia viral vector, a hybrid viral vector, a baculoviral vector, a herpes simplex viral vector, and a lentiviral vector.

In some preferred embodiments, the non-viral vector comprises a plasmid vector.

In a sixth aspect of the present disclosure, provided is a delivery system, wherein the delivery system carries the Cas13 protein of the first aspect, the fusion protein of the second aspect, the CRISPR-Cas system of the third aspect, the polynucleotide of the fourth aspect, or the vector of the fifth aspect.

In some preferred embodiments, the delivery carrier of the delivery system comprises at least one selected from the group consisting of a liposomal carrier, a chitosan carrier, a polymer carrier, a nanoparticle carrier, an exosome carrier, an ectosome carrier, a microbubble carrier, a viral vector, a gene gun, and an electroporation device.

In a seventh aspect of the present disclosure, provided is a host cell, wherein the host cell carries the Cas13 protein of the first aspect, the fusion protein of the second aspect, the CRISPR-Cas system of the third aspect, the polynucleotide of the fourth aspect, or the vector of the fifth aspect.

In an eighth aspect of the present disclosure, provided is a model, wherein the model is a plant model or an animal model; and the plant model or animal model comprises the host cell of the seventh aspect.

In a ninth aspect of the present disclosure, provided is a method for gene editing, wherein the method comprises contacting a target RNA with the Cas13 protein of the first aspect, the fusion protein of the second aspect, the CRISPR-Cas system of the third aspect, the polynucleotide of the fourth aspect, the vector of the fifth aspect, or the host cell of the seventh aspect.

In some preferred embodiments, the guide RNA binds to the Cas13 protein to form a complex, and guides the complex to contact the target RNA, so as to perform gene editing on the target RNA.

In a tenth aspect of the present disclosure, provided is a gene-edited product, wherein the gene-edited product is obtained by performing gene editing on a target RNA using the method of the ninth aspect.

In an eleventh aspect of the present disclosure, provided is use of the Cas13 protein of the first aspect, the fusion protein of the second aspect, the CRISPR-Cas system of the third aspect, the polynucleotide of the fourth aspect, the vector of the fifth aspect, the delivery system of the sixth aspect, the host cell of the seventh aspect, or the method of the ninth aspect in gene editing.

In a twelfth aspect of the present disclosure, provided is a kit, wherein the kit comprises the Cas13 protein of the first aspect, the fusion protein of the second aspect, the CRISPR-Cas system of the third aspect, the polynucleotide of the fourth aspect, the vector of the fifth aspect, the delivery system of the sixth aspect, or the host cell of the seventh aspect.

In a thirteenth aspect of the present disclosure, provided is a nucleic acid detecting method, wherein the method comprises detecting a nucleic acid in a test sample using the kit of the twelfth aspect.

In a fourteenth aspect of the present disclosure, provided is use of the Cas13 protein of the first aspect, the fusion protein of the second aspect, the CRISPR-Cas system of the third aspect, the polynucleotide of the fourth aspect, the vector of the fifth aspect, the delivery system of the sixth aspect, the host cell of the seventh aspect, the kit of the twelfth aspect, or the nucleic acid detecting method of the thirteenth aspect in nucleic acid detection.

In a fifteenth aspect of the present disclosure, provided is use of the Cas13 protein of the first aspect, the fusion protein of the second aspect, the CRISPR-Cas system of the third aspect, the polynucleotide of the fourth aspect, the vector of the fifth aspect, the delivery system of the sixth aspect, or the host cell of the seventh aspect in the manufacture of a medicament for treating a disorder or disease in a subject in need thereof.

In a sixteenth aspect of the present disclosure, provided is a method for treating a disorder or disease in a subject in need thereof, the method comprising: administering to the subject a composition comprising the CRISPR-Cas system of the third aspect of the present disclosure or a polynucleotide encoding the system; wherein there is complementarity of at least 15 nucleotides between the spacer sequence and a target RNA associated with the disease or disorder; wherein the Cas13 protein binds to the guide RNA to form a complex, the complex binds to the target RNA, and the Cas13 protein edits (e.g., cleaves) the target RNA, thereby achieving the purpose of treating the disorder or disease in the subject.

In some preferred embodiments, the disorder or disease is cancer, an infectious disease, or another disease.

In some embodiments, the cancer is Wilms' tumor, Ewing sarcoma, a neuroendocrine tumor, a glioblastoma, a neuroblastoma, a melanoma, skin cancer, breast cancer, colon cancer, rectal cancer, prostate cancer, liver cancer, renal cancer, pancreatic cancer, lung cancer, biliary cancer, cervical cancer, endometrial cancer, esophageal cancer, gastric cancer, head and neck cancer, medullary thyroid carcinoma, ovarian cancer, glioma, lymphoma, leukemia, myeloma, Hodgkin lymphoma, non-Hodgkin lymphoma, or bladder cancer.

In some preferred embodiments, the leukemia includes acute lymphoblastic leukemia (ALL), acute myeloid leukemia (AML), chronic lymphocytic leukemia (CLL), or chronic myeloid leukemia (CML).

In some embodiments, the another disease includes a neurological disease, an otological disease, a muscle abnormality, or an ophthalmological disease.

In some preferred embodiments, the neurological disease includes a neurodevelopmental disorder, Angelman syndrome, or amyotrophic lateral sclerosis (ALS).

In some preferred embodiments, the otological disease includes dominantly inherited deafness.

In some preferred embodiments, the muscle abnormality includes spinal muscular atrophy (SMA).

In some preferred embodiments, the ophthalmological disease includes Leber congenital amaurosis type 2 (LCA2).

In some embodiments, the method is an in vitro method, an in vivo method, or an ex vivo method.

The technical solutions of the present disclosure have the following advantages:

The Cas13 proteins provided by the present disclosure, including Cas13a_10, Cas13a_11, Cas13b_3, Cas13b_4, Cas13b_5, Cas13T_9, Cas13T_15, Cas13T_16, and Cas13Z_2, have a low homology with previously reported Cas13 proteins, and exhibit RNA nuclease activity for editing target RNA. Therefore, Cas13a_10, Cas13a_11, Cas13b_3, Cas13b_4, Cas13b_5, Cas13T_9, Cas13T_15, Cas13T_16, and Cas13Z_2 all have great application prospects in gene editing.

The Cas proteins involved in the present disclosure have collateral cleavage activity and can be used for RNA detection. For example, the Cas13a_10, Cas13a_11, Cas13b_3, Cas13b_4, Cas13b_5, Cas13T_9, Cas13T_15, Cas13T_16, and Cas13Z_2 proteins of the present disclosure exhibit non-specific/ collateral RNase cleavage activity (hereafter referred to as "collateral cleavage activity" for brevity), which can be used to detect the presence of specific RNA, enabling highly sensitive and convenient nucleic acid detection. For instance, they can be widely applied to on-site detection of various medical, biological, and environmental samples, with very broad application prospects.

In addition, Cas13a_10 consists of only 519 amino acids, Cas13a_11 consists of only 726 amino acids, Cas13T_15 consists of 543 amino acids, Cas13T_16 consists of only 845 amino acids, and Cas13Z_2 consists of 849 amino acids. The smaller length of these Cas13 proteins facilitates encapsulation, packaging, and delivery during gene editing; for example, delivery can be achieved using only an AAV vector.

### BRIEF DESCRIPTION OF THE DRAWINGS

FIG. 1 shows a schematic diagram of the genomic locus structure of the CRISPR-Cas system.
FIG. 2 shows the predicted secondary structures of direct repeat (DR) sequences associated with Cas13a_10, Cas13a_11, Cas13b_3, Cas13b_4, and Cas13b_5. Since the DR sequences of Cas13a_10 and Cas13a_11 are identical, the DR sequences of both are named DR-Cas13a_10/11.
FIG. 3 shows the predicted secondary structures of direct repeat (DR) sequences associated with Cas13T_9, Cas13T_15, Cas13T_16, and Cas13Z_2.
FIG. 4 shows a phylogenetic tree of Cas13a_10, Cas13a_11, Cas13b_3, Cas13b_4, Cas13b_5, Cas13T_9, Cas13T_15, Cas13T_16, Cas13Z_2, and previously identified Cas13 proteins.
FIG. 5 shows the conserved domain structures of Cas13a_10, Cas13a_11, Cas13b_3, Cas13b_4, Cas13b_5, Cas13T_15, Cas13T_16, and Cas13Z_2, and indicates the relative positions of the RXXXXH motifs.
FIG. 6 shows the predicted three-dimensional (3D) protein structures of Cas13a_10, Cas13a_11, Cas13b_3, Cas13b_4, Cas13b_5, and Cas13T_9.
FIG. 7 shows the predicted three-dimensional (3D) protein structures of Cas13T_15, Cas13T_16, and Cas13Z_2.
FIG. 8 shows the relationship between the guide RNAs employing different structures and the editing activity of Cas13a_10, Cas13a_11, Cas13b_3, and Cas13b_5 on target RNA (i.e., the knockdown of reporter gene mCherry expression in transfected human HEK293T cells). In the figure, the y-axis represents the relative mean fluorescence intensity, and the x-axis represents different crRNAs.
FIG. 9 shows the relationship between the guide RNAs employing different structures and the editing activity of Cas13b_4, Cas13T_9, and Cas13T_15 on target RNA (i.e., the knockdown of reporter gene mCherry expression in transfected human HEK293T cells). In the figure, the y-axis represents the relative mean fluorescence intensity, and the x-axis represents different crRNAs.
FIG. 10 shows the relationship between the guide RNAs employing different structures and the editing activity of Cas13T_16 and Cas13Z_2 on target RNA (i.e., the knockdown of reporter gene mCherry expression in transfected human HEK293T cells). In the figure, the y-axis represents the relative mean fluorescence intensity, and the x-axis represents different crRNAs.
FIG. 11 shows the relationship between the different spacer sequences and collateral cleavage activity of Cas13b_3, Cas13b_4, and Cas13T_9 on the GFP reporter gene (i.e., the GFP knockdown efficiency in transfected human HEK293T cells represents the non-specific nuclease activity of the aforementioned Cas proteins). In the figure, the y-axis represents the relative mean fluorescence intensity, and the x-axis represents different crRNAs.
FIG. 12 shows the relationship between the different spacer sequences and collateral cleavage activity of Cas13T_15 and Cas13T_16 on the GFP reporter gene (i.e., the GFP knockdown efficiency in transfected human HEK293T cells represents the non-specific nuclease activity of the aforementioned Cas proteins). In the figure, the y-axis represents the relative mean fluorescence intensity, and the x-axis represents different crRNAs.
FIG. 13 shows the plasmid map of U6-BbSI-PUC57, which is used for constructing guide RNA recombinant plasmids.
FIG. 14 shows a comparison of the cleavage activity of Cas13T_9, Cas13Z_2, Cas13T_15, Cas13T_16, Cas13b_3, and Cas13d at specific sites.
FIG. 15 shows the knockdown effect of Cas13T_15 on the mRNA expression of various endogenous genes.

### DETAILED DESCRIPTION

Embodiments of the present disclosure relate to Cas13 proteins.

Cas13 proteins are a class of proteins capable of cleaving single-stranded RNA. The present disclosure relates to nine novel Cas13 proteins, including:
proteins having an amino acid sequence represented by any one of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, or SEQ ID NO. 9; and
functional active variants thereof, i.e., proteins or polypeptides whose amino acid sequences are not completely identical to any one of SEQ ID NOs. 1-9 but have the same activity or substantially the same activity as the latter. For example, when calculated based on the activity characterized in the examples, the activity is 0.3-fold, 0.4-fold, 0.5-fold, 0.6-fold, 0.7-fold, 0.8-fold, 0.9-fold, 1.0-fold, 1.2-fold, 1.5-fold, 1.8-fold, 2-fold, etc., of that of any one of SEQ ID NOs. 1-9.

It is well known to those skilled in the art that amino acid changes (e.g., substitutions, deletions, or additions) may occur in Cas13 proteins, and the resulting Cas13 proteins can still retain their functions or activities.

The term "substitution" refers to the replacement of one amino acid residue at a specific position in the amino acid sequence with another amino acid residue; among them, the "substitution" may be a conservative amino acid substitution.

"Conservative modification", "conservative substitution", or "conservative replacement" refers to the replacement of an amino acid in a protein with another amino acid having similar characteristics (e.g., charge, side-chain size, hydrophobicity/hydrophilicity, backbone conformation, rigidity, etc.), which enable that such changes can be made frequently without altering the biological activity of the protein.

It is known to those skilled in the art that, in general, a single amino acid substitution in a non-essential region of a polypeptide does not substantially alter the biological activity (see Watson et al. (1987), Molecular Biology of the Gene, The Benjamin/Cummings Pub. Co., p. 224, (4th ed.)). In addition, substitution of amino acids with similar structures or functions is unlikely to disrupt biological activity. Exemplary conservative substitutions are set forth in "Exemplary Conservative Amino Acid Substitutions" (see Table 1).

**Table 1. Exemplary Conservative Amino Acid Substitutions**

| Original Residue | Conservative Substitutions |
|---|---|
| Ala(A) | Gly(G); Ser(S) |
| Arg(R) | Lys(K); His(H) |
| Asn(N) | Gln(Q); His(H); Asp(D) |
| Asp(D) | Glu(E); Asn(N) |
| Cys(C) | Ser(S); Ala(A); Val(V) |
| Gln(Q) | Asn(N); Glu(E) |
| Glu(E) | Asp(D); Gln(Q) |
| Gly(G) | Ala(A) |
| His(H) | Asn(N); Gln(Q) |
| Ile(I) | Leu(L); Val(V) |
| Leu(L) | Ile(I); Val(V) |
| Lys(K) | Arg(R); His(H) |
| Met(M) | Leu(L); Ile(I); Tyr(Y) |
| Phe(F) | Tyr(Y); Met(M); Leu(L) |
| Pro(P) | Ala(A) |
| Ser(S) | Thr(T) |
| Thr(T) | Ser(S) |
| Trp(W) | Tyr(Y); Phe(F) |
| Tyr(Y) | Trp(W); Phe(F) |
| Val(V) | Ile(I); Leu(L) |

Embodiments of the present disclosure relate to fusion proteins comprising the Cas13 protein of the present disclosure. Preferred embodiments of the present disclosure relate to fusion proteins comprising the Cas13 protein of the present disclosure and a heterologous functional domain.

In some embodiments of the present disclosure, the heterologous functional domain of the fusion protein comprises at least one selected from the group consisting of a deaminase, a reporter protein, a detection label, a localization signal, a protein targeting domain, a DNA-binding domain, an epitope tag, a transcriptional activation domain, a transcriptional repression domain, a nuclease, a methylation-active enzyme, a demethylase, a transcript release factor, an RNA-cleaving polypeptide, a nucleic acid ligase, a histone modification activator, and a nucleic acid-binding activator.

The fusion protein of the present disclosure, obtained by fusing the Cas13 protein of the present disclosure with a heterologous functional domain, can achieve specific detection, mutation, or therapeutic purposes through the combination of two or more protein functional activities, such as those described below.

In some embodiments, the fusion protein comprising the Cas13 protein of the present disclosure can utilize a negative feedback regulatory mechanism to improve the signal-to-noise ratio by means of a nuclear localization sequence (NLS), a zinc finger protein, and a KRAB domain.

In some embodiments, the fusion protein comprising the Cas13 protein of the present disclosure can achieve site-specific RNA methylation and/or site-specific RNA demethylation.

In some embodiments, the fusion protein comprising the Cas13 protein of the present disclosure can target pathogenic tau pre-mRNA, alter alternative splicing patterns, and achieve a significant therapeutic effect.

Embodiments of the present disclosure relate to CRISPR-Cas systems comprising the Cas13 protein of the present disclosure.

The CRISPR-Cas system of the present disclosure comprises the Cas13 protein of the present disclosure and a guide RNA, or comprises the fusion protein of the present disclosure and a guide RNA.

In some embodiments of the present disclosure, the guide RNA is capable of binding to the Cas13 protein to form a complex and guiding the complex to contact a target RNA.

The guide RNA of the present disclosure comprises a spacer sequence and a direct repeat sequence; the spacer sequence is between 15-60 nucleotides, e.g., 20-50 nucleotides, 30-40 nucleotides, or 30 nucleotides; the direct repeat (DR) sequence is between 15-40 nucleotides, e.g., 20-35 nucleotides, 25-30 nucleotides, or 30 nucleotides.

In some embodiments, the guide RNA is chemically modified; for example, 2'-O-methyl, 2'-O-methyl-3'-thiophosphate, or 2'-O-methyl-3'-thioPACE is incorporated at one or more terminal nucleotides. Compared with unmodified guide RNAs, the above chemically modified guide RNAs are more stable and also have increased activity.

In some embodiments, the spacer sequence has complementarity to at least 15 nucleotides of the target RNA, e.g., 15, 16, 18, 19, 20, or 21-30 nucleotides.

The CRISPR-Cas system of the present disclosure can be used for nucleic acid detection. In some embodiments of the present disclosure, after the CRISPR-Cas13 system binds to a crRNA and a substrate RNA to form a ternary complex, the Cas13 protein is activated. At this point, the Cas13 protein can not only specifically cleave the substrate RNA but also cleave any single-stranded RNA free in the environment. This phenomenon is referred to as non-specific nuclease activity, collateral activity, or collateral cleavage. Utilizing this property, the system of the present disclosure can detect RNA; for example, the detection sensitivity can reach the femtomolar level within 10-15 minutes. Compared with quantitative polymerase chain reaction (qPCR), which is the traditional gold standard for nucleic acid detection, the detection of the present disclosure is faster and lower equipment dependency.

The CRISPR-Cas system of the present disclosure can be used for RNA imaging. In some embodiments of the present disclosure, the Cas13 protein is mutated to obtain a protein variant that loses endonuclease activity but retains binding activity, thereby enabling targeting of target RNA molecules.

The CRISPR-Cas system of the present disclosure can be used for transcriptome regulation. In some embodiments of the present disclosure, fusing various effector proteins to the Cas13 protein or its protein variant (which loses endonuclease activity but retains binding activity) enables various types of regulation targeting RNA. Examples include methylation regulation and treatment of genetic diseases caused by single-base mutations.

By fusing a specific mutant of the Cas13 protein of the present disclosure (e.g., a mutant that loses part or all of its nuclease activity) with a deaminase (e.g., ADAR2DD, etc.), an RNA base editor can be constructed.

In some embodiments, the CRISPR-Cas system of the present disclosure can achieve therapeutic purposes by targeting overexpressed RNA, or existing toxic RNA, or mutant RNA (e.g., RNA with splicing defects or truncations, whose overexpression causes diseases). Alternatively, the CRISPR-Cas system of the present disclosure can also be used for treatment by targeting transacting mutant sequences that mediate RNA-dependent functions causing diseases. For example, mutations in the SNHG5 gene are associated with various cancers (e.g., prostate cancer); and targeting the RNA expressed by SNHG5 can provide a therapeutic approach for r related diseases.

Embodiments of the present disclosure relate to polynucleotides.

The polynucleotide of the present disclosure encodes the Cas13 protein of the present disclosure, or the polynucleotide encodes the fusion protein of the present disclosure, or the polynucleotide encodes the CRISPR-Cas system of the present disclosure.

In some embodiments, the aforementioned polynucleotide is a DNA molecule. In other embodiments, the polynucleotide is an RNA molecule (e.g., an mRNA molecule encoding the Cas13 protein, a derivative thereof, or a functional fragment thereof). In some embodiments, the aforementioned RNA molecule (e.g., mRNA molecule) is capped, polyadenylated, or substituted with 2'-O-methyl analogs, 2'-deoxy analogs, 2-thiouridine analogs, N6-methyladenosine analogs, 2'-fluoro analogs, 5-methylcytidine, pseudouridine, or any combination thereof. The substitutions include, but are not limited to, modifications with 2-aminopurine, 5-bromo-uridine, pseudouridine (Ψ), N1-methylpseudouridine (me1Ψ), 5-methoxyuridine (5moU), inosine, 7-methylguanosine, and the like.

In some embodiments, the polynucleotide has at least 50%, 60%, 75%, 80%, 85%, 90%, 95%, 97.5%, 99%, or 100% nucleotide sequence identity to the nucleotide sequence disclosed in the present disclosure.

In some embodiments, the amino acid sequence encoded by the polynucleotide has at least 85% (e.g., 85%, 95%, 99%) sequence identity to the amino acid sequence set forth in any one of SEQ ID NOs. 1-9.

Embodiments of the present disclosure relate to vectors, which are capable of delivering or introducing the Cas13 protein (including homologs thereof) and/or guide RNA involved in the present disclosure into host cells, and in some specific cells (e.g., prokaryotic cells), are also capable of replicating these components (e.g., in prokaryotic cells). When operably linked to appropriate regulatory elements, the vectors are capable of replication. The aforementioned vectors include a variety of types, with common ones being viral vectors and non-viral vectors. One type of vector is a viral vector, in which virus-derived DNA or RNA sequences are present in the vector used for packaging viruses (e.g., retroviral vectors, phage vectors, adenoviral vectors, adeno-associated viral vectors, vaccinia viral vectors, hybrid viral vectors, baculoviral vectors, herpes simplex viral vectors, or lentiviral vectors). Viral vectors often include polynucleotides carried by viruses for transfection into host cells. In addition, a common type of non-viral vector is a plasmid, which is a circular double-stranded DNA loop into which additional DNA segments can be inserted, for example, via standard molecular cloning techniques. Certain plasmid vectors (e.g., bacterial vectors with a bacterial origin of replication and episomal mammalian vectors) are capable of autonomous replication in the host cells into which they are introduced.

The vector of the present disclosure carries the polynucleotide described above, e.g., a polynucleotide encoding the Cas13 protein of the present disclosure, or encoding the aforementioned fusion protein of the Cas13 protein, or encoding the CRISPR-Cas system of the present disclosure.

The polynucleotide further includes a nucleotide sequence encoding the direct repeat (DR) sequence set forth in SEQ ID NOs. 20-27.

In some embodiments of the present disclosure, the polynucleotide carried by the vector is linked with regulatory elements.

In some embodiments of the present disclosure, the regulatory elements include at least one selected from the group consisting of a promoter, an internal ribosomal entry site (IRES), an enhancer, a polyadenylation signal, a terminator, a protein degradation signal, a transposon, a leader sequence, a polyadenylic acid sequence, and a marker gene.

Promoters include tissue-specific promoters, which can direct expression primarily in a tissue of interest (such as muscle, neuron, bone, skin, blood), a specific organ (e.g., liver, pancreas), or a specific cell type (e.g., lymphocyte). Specifically, the promoter may comprise a U6 promoter, an H1 promoter, a retroviral Rous sarcoma virus (RSV) LTR promoter (optionally with an RSV enhancer), a cytomegalovirus (CMV) promoter (optionally with a CMV enhancer), an SV40 promoter, a dihydrofolate reductase promoter, a β-actin promoter, a phosphoglycerate kinase (PGK) promoter, and an EF1α promoter.

Enhancer elements include a CMV enhancer, an RSV enhancer, an SV40 enhancer, and the like. When designing an expression vector, factors such as the host cell to be transformed and the expected expression level may be considered. The vector can be introduced into a host cell to produce transcripts, proteins, or peptides encoded by the nucleic acids described herein, including fusion proteins or peptides (e.g., clustered regularly interspaced short palindromic repeats (CRISPR) transcripts, proteins, enzymes, mutant forms thereof, fusion proteins thereof, etc.).

Embodiments of the present disclosure relate to delivery systems. The delivery system of the present disclosure is used to deliver the protein, nucleic acid, system, or vector of the present disclosure into a body for detection and treatment.

In some embodiments of the present disclosure, the delivery system carries the Cas13 protein of the present disclosure, the fusion protein of the present disclosure, the CRISPR-Cas system of the present disclosure, the polynucleotide of the present disclosure, or the vector of the present disclosure.

In some embodiments of the present disclosure, the delivery carrier of the delivery system includes at least one selected from the group consisting of liposomal carriers, chitosan carriers, polymer carriers, nanoparticle carriers, exosome carriers, ectosome carriers, microbubble carriers, viral vectors, gene guns, and electroporation devices.

In addition, when the delivery target is certain cells (e.g., plant cells), the delivery methods using, for example, cell-penetrating peptides (CPPs) may also be employed. For example, in one specific embodiment, the Cas protein and/or at least one guide RNA is conjugated with one or more CPPs, thereby effectively transporting the CPPs conjugated with the Cas protein and/or guide RNA into plant cells (e.g., into protoplasts). CPPs are short peptides with fewer than 35 amino acids, derived from proteins or chimeric sequences, and capable of transporting biomolecules across cell membranes in a receptor-independent manner. CPPs can be cationic peptides, peptides with hydrophobic sequences, amphipathic peptides, peptides rich in proline and antimicrobial sequences, and chimeric or bipartite peptides. CPPs can penetrate biological membranes and thus trigger the movement of different biomolecules across cell membranes into the cytoplasm, improve their intracellular pathways, and thereby facilitate the interaction of biomolecules with targets.

In exemplary cases, CPPs include Tat (a nuclear transcription activation protein required for viral replication by HIV type 1), penetratin, Kaposi fibroblast growth factor (FGF) signal peptide sequence, integrin β3 signal peptide sequence, polyarginine peptide (Arg sequence), guanine-rich molecular transporters, sweet arrow peptide, and the like.

Embodiments of the present disclosure relate to host cells, wherein the host cell carries the Cas13 protein of the present disclosure, the fusion protein of the present disclosure, the CRISPR-Cas system of the present disclosure, the polynucleotide of the present disclosure, or the vector of the present disclosure.

In some embodiments, the host cell is an ex vivo, in vivo, or in vitro cell, or a cell line or progeny thereof, wherein the cell, cell line, or progeny comprises: the aforementioned Cas13 protein, fusion protein, CRISPR-Cas system, polynucleotide, vector, or delivery system.

In some embodiments, the cell is a prokaryotic cell, e.g., Escherichia coli.

In some embodiments, the cell is a eukaryotic cell. In some embodiments, the cell is a yeast cell, a plant cell, or a mammalian cell (including human and non-human mammalian cells). In some embodiments, the cell is a non-human mammalian cell, e.g., a cell from a non-human primate, cow, sheep, pig, dog, monkey, rabbit, or rodent (such as a rat or mouse). In some embodiments, the cell is a non-mammalian eukaryotic cell, e.g., a cell from a bird (such as a chicken), fish, or crustacean. In some embodiments, the cell is a plant cell, e.g., a cell from a monocotyledonous plant, a dicotyledonous plant, a cultivated plant, or a food crop (such as corn, sorghum, soybean, wheat, oat, or rice). In some embodiments, the cell is a stem cell or a stem cell line. In certain cases, the host cell of the present disclosure comprises a modification to a gene or genome, which is a modification not present in its wild-type form.

In some embodiments, the host cell is selected from the group consisting of Escherichia coli, wheat germ cells, insect cells, SF9 cells, Hela cells, HEK293 cells, CHO cells, yeast cells, or a combination thereof.

Embodiments of the present disclosure relate to models.

In some embodiments of the present disclosure, the model comprises the host cell of the present disclosure.

Embodiments of the present disclosure relate to methods for gene editing.

In some embodiments of the present disclosure, the method comprises contacting a target RNA with the Cas13 protein of the present disclosure, the fusion protein of the present disclosure, the CRISPR-Cas system of the present disclosure, the polynucleotide of the present disclosure, or the vector of the present disclosure. In some embodiments of the present disclosure, the contacting enables editing by the Cas13 protein.

In some embodiments of the present disclosure, in the method, the guide RNA binds to the Cas13 protein to form a complex, and guides the complex to contact the target RNA, so as to perform gene editing on the target RNA.

Embodiments of the present disclosure relate to gene-edited products. In some embodiments of the present disclosure, the gene-edited product is obtained by performing gene editing on a target RNA using the gene editing method of the present disclosure.

Therefore, embodiments of the present disclosure relate to use in gene editing.

Embodiments of the present disclosure relate to kits.

In some embodiments of the present disclosure, the kit comprises the Cas13 protein of the present disclosure, the fusion protein of the present disclosure, the CRISPR-Cas system of the present disclosure, the polynucleotide of the present disclosure, the vector of the present disclosure, the delivery system of the present disclosure, or the host cell of the present disclosure. In some embodiments, the kit further comprises instructions for using the kit, e.g., instructions in one or more languages. The kit may further comprise one or more reagents for use in processes utilizing one or more of the aforementioned components. The reagents may be provided in any suitable container. For example, the kit may provide one or more reaction or stock buffers. The aforementioned reagents may be provided in a form requiring the addition of one or more other components before use (e.g., in concentrated or lyophilized form); the buffers may be any buffers, including but not limited to sodium carbonate buffers, sodium bicarbonate buffers, borate buffers, Tris buffers, MOPS buffers, HEPES buffers, and combinations thereof. The buffers may have a suitable pH, e.g., may be alkaline. In some embodiments, the pH of the buffer is between about 7-10.

Embodiments of the present disclosure relate to nucleic acid detecting methods. In some embodiments of the present disclosure, the nucleic acid detecting method of the present disclosure is completed by detecting a nucleic acid in a test sample using the kit of the present disclosure, or using one or more components contained therein (e.g., the Cas13 protein, fusion protein, CRISPR-Cas system, polynucleotide, vector, delivery system, or host cell).

In some embodiments, the CRISPR-Cas system of the present disclosure can be used for DNA and/or RNA detection. For example, as shown in the examples, when the spacer sequence is about 30 nucleotides and activated by its guide RNA-dependent specific RNase activity, Cas13b_3, Cas13b_4, Cas13T_9, Cas13T_15, and Cas13T_16 of the present disclosure exhibit collateral cleavage activity (non-specific cleavage activity). Therefore, the Cas13 protein involved in the present disclosure enables sample nucleic acid detection by reprogramming the guide RNA (crRNA), thereby achieving specific RNA detection.

For example, the CRISPR-Cas system of the present disclosure can be used in the SHERLOCK method (for details, see, e.g., Gootenberg et al., "Nucleic acid detection with CRISPR-Cas13a/C2c2", Science, 356(6336): 438-442 (2017)), which is incorporated herein by reference in its entirety.

The embodiments of the present disclosure relate to use in nucleic acid detection, or the embodiments of the present disclosure relate to use in the manufacture of a medicament for a condition or disease.

In some embodiments of the use of the present disclosure, the aforementioned nucleic acid detection is performed using the kit of the present disclosure, or using one or more components contained therein, e.g., the Cas13 protein, fusion protein, CRISPR-Cas system, polynucleotide, vector, delivery system, or host cell.

Unless otherwise defined, all technical and scientific terms used herein have the meanings commonly understood by those skilled in the art to which the present disclosure pertains. As used herein, the following terms have the meanings ascribed to them below, unless otherwise specified.

"Polynucleotide" and "nucleic acid" refer to a polymeric form of nucleotides (e.g., RNA or DNA) of any length. The term includes, but is not limited to, single-stranded, double-stranded, or multi-stranded DNA or RNA, genomic DNA, cDNA, DNA-RNA hybrids, or polymers containing purine and pyrimidine bases or other naturally occurring, chemically or biochemically modified, non-natural, or derived nucleotide bases.

"A DNA sequence encoding a specific RNA" refers to a DNA nucleotide sequence that is transcribed into RNA.

"Regulatory element" or " control element" refers to transcriptional and translational regulatory sequences, such as promoters, enhancers, terminators, and the like.

"Promoter (also referred to as a promoter sequence)" is a DNA regulatory region capable of binding RNA polymerase and initiating transcription of downstream (3' direction) coding or noncoding sequences. In general, various promoters, including inducible promoters, can be used to drive the expression of the various vectors of the present disclosure.

"Codon optimization" refers to modification of a nucleic acid sequence to enable better expression in a host cell, typically by replacing at least one codon (e.g., one or more, such as 10, 20, or more) in the original nucleic acid sequence with a codon that is more frequently or most frequently used in the genes of the host cell, while maintaining the expressed amino acid sequence as the native one.

"Target RNA" can be any target RNA molecule, which may be a naturally occurring RNA molecule or an engineered RNA molecule (e.g., mRNA, tRNA, ribosomal RNA (rRNA), microRNA (miRNA), small interfering RNA (siRNA), ribozyme, satellite RNA, or viral RNA, etc.). The target RNA may be a target RNA associated with a condition or disease (e.g., an infectious disease or cancer). Perfect complementarity between the target RNA and the guide RNA is not required, as long as sufficient complementarity exists to cause hybridization and promote CRISPR-Cas complex formation.

"Naturally occurring (also referred to as unmodified, non-modified, wild-type (wt))" refers to a nucleic acid, polypeptide, cell, or organism that exists in nature. For example, a polypeptide or polynucleotide sequence that exists in an organism and can be isolated from a source in nature is naturally occurring.

"Fusion protein" refers to a chimeric polypeptide containing protein domains from at least two different proteins. One protein may be located in the amino-terminal (N-terminal) portion or the carboxy-terminal (C-terminal) portion of the fusion protein, thereby forming an amino-terminal fusion protein or a carboxy-terminal fusion protein, respectively.

"CRISPR-Cas system" (also referred to as "CRISPR system") generally includes transcripts or other elements involved in the expression of CRISPR-associated ("Cas") genes, or transcripts or other elements capable of directing the activity of the Cas genes. In some embodiments, components of the CRISPR system may include nucleic acids (e.g., vectors) encoding one or more components of the system, components in protein form, or a combination thereof.

"Cas protein" refers to a CRISPR-associated protein (Cas) (also referred to as "CRISPR-associated protein", "CRISPR effector", "effector", "Cas protein", "Cas enzyme", or "CRISPR enzyme") that performs enzymatic activity and/or binds to a target site on a nucleic acid specified by an RNA guide. In some embodiments, the Cas protein has endonuclease activity, nickase activity, exonuclease activity, transposase activity, and/or excision activity; in other embodiments, the Cas protein may be nuclease-inactivated.

"Guide RNA (also referred to as guiding RNA, gRNA, or crRNA)" refers to any RNA molecule that facilitates the targeting of the Cas13 protein of the present disclosure to a target nucleic acid (such as DNA and/or RNA).

In the CRISPR-Cas system, the degree of complementarity between the guide RNA and its corresponding target nucleic acid may not be 100%, and may be e.g., about 50%, 55%, 60%, 75%, 80%, 85%, 90%, 95%, 97.5%, 99%. To reduce off-target interactions, e.g., to reduce interactions between the guide RNA and target nucleic acids with low complementarity, mutations can be introduced into the CRISPR-Cas system, enabling it to distinguish between target nucleic acids having complementarity greater than 80%, 85%, 90%, 95%, 99% and off-target sequences. In some specific embodiments, the degree of hybridization/complementarity between the guide RNA and the corresponding target nucleic acid is from 80% to 95% (e.g., there is about 83%, 84%, 85%, 86%, 87%, 88%, 89%, 90%, 91%, 92%, 93%, 94%, or 95% complementarity). Thus, in some embodiments, the degree of complementarity between the guide RNA and its corresponding target sequence is greater than 93%, 93.5%, 95%, 95.5%, 96%, 96.5%, 97%, 97.5%, 98%, 98.5%, 99%, 99.5%, or 99.9%. In some embodiments, the degree of complementarity is 100%.

It is known in the art that a complete complementarity is not required, provided that the existing complementarity is sufficient to function. Thus, if desired, a modulation to cleavage efficiency can be achieved by introducing mismatches (e.g., one or more mismatches between the spacer sequence and the target nucleic acid, such as mismatches of 1 or 2 nucleotides (including positions of mismatches along the spacer/target sequence)). The cleavage efficiency is more affected if the mismatch (e.g., a double mismatch) is located closer to the center (i.e., not at the 3' or 5' end). Thus, cleavage efficiency can be modulated by selecting the position of the mismatch along the spacer sequence. For example, if less than 100% cleavage of the target is desired (e.g., in a cell population), 1 or 2 mismatches between the spacer sequence and the target sequence can be introduced into the spacer sequence.

"Vector" refers to a polynucleotide composition used to transfer, deliver, or introduce a nucleic acid into a host cell. Suitable vectors include plasmid vectors, phage vectors, viral vectors (e.g., retroviral vectors, adeno-associated viral vectors, herpes simplex viral vectors, AAV vectors, lentiviral vectors, baculoviral vectors), and the like.

"Delivery system" includes a delivery carrier, which includes one or more liposomes, nanoparticles, exosomes, microbubbles, viral vectors, or gene guns.

In some embodiments, a vector (e.g., a viral or non-viral vector, such as a lentiviral vector or plasmid) can be delivered to a target tissue by, for example: intramuscular injection, intravenous administration, transdermal administration, intranasal administration, oral administration, or mucosal administration. The aforementioned delivery can be via a single dose or multiple doses. It will be understood by those skilled in the art that the actual dose to be delivered herein can vary considerably depending on a variety of factors, including but not limited to the choice of vector, target cell, organism, tissue, general condition of the subject to be treated, the degree of transformation/modification sought, route of administration, mode of administration, and the type of transformation/modification sought.

"Functional fragment" refers to a protein or polypeptide sequence that includes fewer amino acids than the original sequence of the protein or polypeptide, but the remaining amino acid sequence retains a certain proportion (e.g., 10%, 20%, 30%, 40%, 50%, or 60-99%, 100%) of the functional activity relative to the functional activity of the original reference sequence (e.g., a protein or polypeptide can be modified by substitution, insertion, deletion, and/or addition of one or more amino acids while retaining a certain proportion of enzymatic activity).

"Identity" refers to matching sequences between two polypeptides or two nucleic acids. When a position in each of the two sequences being compared is occupied by the same base or amino acid monomer subunit (e.g., if a position in each of two DNA molecules is occupied by adenine, or a position in each of two polypeptides is occupied by lysine), the molecules are identical at that position. The "percent identity" between two sequences is a function of the number of matching positions shared by the two sequences divided by the number of positions compared and multiplied by 100. For example, if 6 out of 10 positions in two sequences match, the two sequences have 60% identity. For example, the DNA sequences CTGACT and CAGGTT share 50% identity (3 out of 6 total positions match). Generally, comparisons are made when the two sequences are aligned to yield maximum identity.

"Hybridization" or "complementary" or "substantially complementary" means that a nucleic acid (e.g., RNA, DNA) contains a nucleotide sequence that enables it to non-covalently bind (i.e., form Watson-Crick base pairs and/or G/U base pairs), "anneal", or "hybridize" with another nucleic acid in a sequence-specific, antiparallel manner (i.e., the nucleic acid specifically binds to a complementary nucleic acid) under appropriate in vitro and/or in vivo temperature and solution ionic strength conditions. Hybridization requires that the two nucleic acids contain complementary sequences, but mismatches between bases are possible. Typically, hybridizable nucleic acids are 8 nucleotides or more in length (e.g., 10 nucleotides or more, 12 nucleotides or more, 15 nucleotides or more, 20 nucleotides or more, 22 nucleotides or more, 25 nucleotides or more, or 30 nucleotides or more).

"Direct repeat (DR) sequence (also referred to as a DR sequence)" refers to a DNA coding sequence in a CRISPR locus, or the RNA encoded thereby in a guide RNA, where each T in the DNA coding sequence is understood to represent a U when described at the RNA level.

The direct repeat sequence comprises a nucleotide sequence that is at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) identical to the nucleotide sequence set forth in any one of SEQ ID NOs. 20-27.

"Host cell" includes in vitro, ex vivo, or in vivo cells or cell lines or their progeny, which contain: the Cas13 protein, fusion protein, CRISPR-Cas composition, recombinant vector, or delivery carrier of the present disclosure.

In some embodiments, the CRISPR-Cas system provided by the present disclosure can be used in prokaryotic or eukaryotic cells. Exemplary prokaryotic cells include Escherichia coli. Eukaryotic cells include, but are not limited to, plant cells, fungal cells (e.g., yeast cells, exemplarily such as Pichia pastoris, Kluyveromyces, Kluyveromyces lactis, etc.), mammalian cells, reptilian cells, insect cells, avian cells, fish cells, parasite cells, arthropod cells, invertebrate cells, vertebrate cells, rodent cells, mouse cells, rat cells, primate cells, non-human primate cells, or human cells. In some embodiments, the eukaryotic cells are in in vitro culture. In some embodiments, the eukaryotic cells are in vivo. In some embodiments, the eukaryotic cells are ex vivo (in vitro).

The following examples are provided for a better further understanding of the present disclosure, and are not limited to the described preferred embodiments, nor do they limit the content and protection scope of the present disclosure. Any product derived by anyone under the inspiration of the present disclosure or by combining the present disclosure with features of other prior art that is identical or similar to the present disclosure shall fall within the protection scope of the present disclosure.

For the following examples where specific experimental procedures or conditions are not indicated, they can be carried out in accordance with the operations or conditions of conventional experimental procedures described in the literature in the art. Reagents or instruments used without indicating the manufacturer are all conventional reagent products available commercially.

### Example 1. Identification of Novel Cas13 Proteins

The inventors identified nine novel Cas13 proteins through metagenomic analysis, de-redundancy (removal of identical protein sequences), and protein clustering analysis. Through analysis, it was found that these proteins belong to four families of Cas13 protein: Cas13a, Cas13b, Cas13T, and Cas13Z families (among them, the Cas13T and Cas13Z families are different from all previously identified Cas13 proteins and belong to brand-new Cas13 families). The nine novel Cas13 proteins include Cas13a_10 (SEQ ID NO.1), Cas13a_11 (SEQ ID NO.2), Cas13b_3 (SEQ ID NO.3), Cas13b_4 (SEQ ID NO.4), Cas13b_5 (SEQ ID NO.5), Cas13T_9 (SEQ ID NO.6), Cas13T_15 (SEQ ID NO.7), Cas13T_16 (SEQ ID NO.8), and Cas13Z_2 (SEQ ID NO.9).

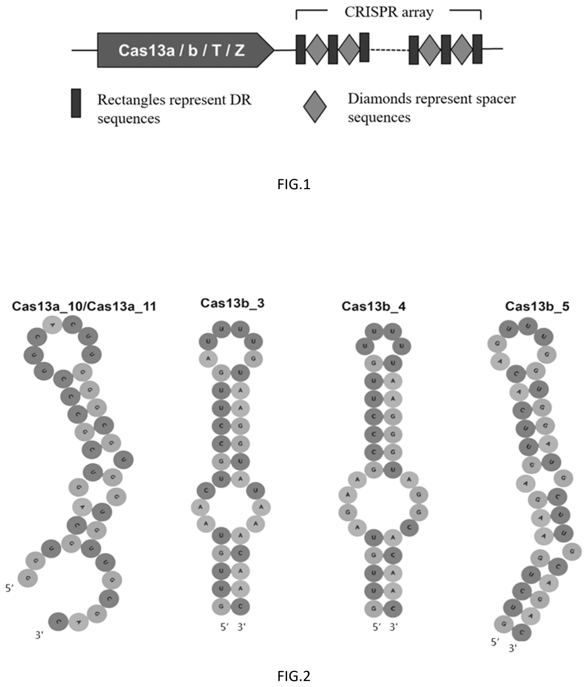

Through analysis, it was found that the genomic locus structures of the above four Cas13 families are as shown in FIG. 1. The components in the locus are not drawn to scale. As shown in the figure, the pointed bar segments labeled "Cas12a/b/T/Z" represent Cas coding sequences, the rectangle segments represent DR sequences, and the diamond segments represent spacer sequences. Comparison showed that these nine Cas13 proteins have low similarity to the previously identified Cas13 proteins.

Through sequence analysis, the direct repeat (DR) sequences associated with the above Cas13 proteins were identified. The DNA sequences encoding the direct repeat (DR) sequences are shown in Table 2.

**Table 2 Coding Sequences of Direct Repeat (DR) Sequences Associated with Different Cas13 Proteins**

| Cas13 Protein | Coding Sequence of Direct Repeat (DR) Sequence | Name |
|---|---|---|
| Cas13a_10 | GGTGCAGTCGCCCTTCACTTGGGCGTGGTGTTGCGAC( SEQ ID NO.20) | DR-Cas13a_10/11 |
| Cas13a_11 | | |
| Cas13b_3 | GTTGTAACTGCCTTGATTTTGTAAGGTATAAACAAC(SE Q ID NO.21) | DR-Cas13b_3 |
| Cas13b_4 | GTTGTAGAAGCCCTTGTTTTTAAGGGTAGGCACAAC(S EQ ID NO.22) | DR-Cas13b_4 |
| Cas13b_5 | GTCTGAAGAGTTCACAGTTTGGTGGATGCTTGCAGAC( SEQ ID NO.23) | DR-Cas13b_5 |
| Cas13T_9 | GTTGTTTATACCTTACAAAATCAAGGCAGTTACAAC(SE Q ID NO.24) | DR-Cas13T_9 |
| Cas13T_15 | GCTGTAACTACCTTTCAAAATCAAGGCTTCCACAGC(SE Q ID NO.25) | DR-Cas13T_15 |
| Cas13T_16 | GTTGTAGCTGTCTTTATTTGAAAGGCACAGACAAC(SE Q ID NO.26) | DR-Cas13T_16 |
| Cas13Z_2 | GCCGTTCCCACCTTACAAACGCAAGGCTTCCACAGCT( SEQ ID NO.27) | DR-Cas13Z_2 |

The secondary structures of the direct repeat (DR) sequences associated with Cas13a_10, Cas13a_11, Cas13b_3, Cas13b_4, Cas13b_5, Cas13T_9, Cas13T_15, Cas13T_16, and Cas13Z_2 were analyzed using the RNA secondary structure prediction software RNAfold. The results of the structural analysis are shown in FIGS. 2 to 3. As can be seen from FIGS. 2 to 3, all DR sequences have relatively conserved secondary structures.

The DR sequences of the two proteins (Cas13a_10 and Cas13a_11) belonging to the Cas13a family are exactly identical. As observed from their secondary structures, both of them form a stem composed of 3 base pairs (5'-GCA-3' and its complementary bases), followed by two small bulges, then a stem composed of 5 base pairs (5'-CGCCC-3' and its complementary bases), and finally a loop structure formed by 7 bases (5'-UUCACUU-3').

Among the three proteins of the Cas13b family, the secondary structures of the DR sequences of Cas13b_3 and Cas13b_4 are highly similar: both form a stem composed of 5 base pairs (5'-GUUGU-3' and its complementary bases), followed by a bulge structure (the type and number of bases of this bulge structure differs: for Cas13b_3, the bulge structure formed at this position of the associated DR sequence consists of 3+3 bases; for Cas13b_4, the bulge formed at this position of its associated DR sequence consists of 4+4 bases), then a stem structure (the type and number of bases of this stem structure differ: for Cas13b_3, the stem structure formed at this position of the associated DR sequence consists of 5'-UGCCUUG-3' and its complementary bases; for Cas13b_4, the stem formed at this position of its associated DR sequence consists of 5'-GCCCUUG-3' and its complementary bases), and finally a loop structure (the type and number of bases of this loop structure differ: for Cas13b_3, the bulge structure formed at this position of the associated DR sequence consists of 6 bases; for Cas13b_4, the bulge formed at this position of its associated DR sequence consists of 4 bases). In contrast, the secondary structure of the DR sequence associated with Cas13b_5 differs significantly from those of Cas13b_3 and Cas13b_4. As shown in FIG. 2, it has 3 stem structures composed of 5 base pairs, 3 base pairs, and 6 base pairs, respectively. Bulge structures exist between the 1st and 2nd stems, and between the 2nd and 3rd stems; a loop structure composed of 6 bases is also present at the end.

In the Cas13T family, the secondary structures of the DR sequences associated with Cas13T_9, Cas13T_15, and Cas13T_16 are similar, each containing a stem structure composed of 5 base pairs (wherein the corresponding segments of Cas13T_9 and Cas13T_16 are identical, both consisting of 5'-GUUGU-3' and its complementary bases, while the base types forming the base pairs of this stem structure in Cas13T_15 are different), followed by a bulge structure composed of multiple bases (among them, the bulge segment in the DR secondary structures associated with Cas13T_9 and Cas13T_15 consists of 5+5 bases, while the bulge structure in the DR secondary structure associated with Cas13T_16 consists of 3+3 bases with differences in base types as well), then another stem structure (wherein the stem segments in the DR secondary structures associated with Cas13T_9 and Cas13T_15 are identical, both consisting of 4 base pairs (5'-CCUU-3') and its complementary bases, while the stem structure in the DR secondary structure associated with Cas13T_16 consists of 7 base pairs (5'-UGUCUUU-3') and its complementary bases), and finally a terminal loop structure (the loop structure in the DR secondary structures associated with Cas13T_9 and Cas13T_15 consists of 8 bases, with only one base differing and the rest being identical, while the loop structure in the DR secondary structure associated with Cas13T_16 consists of 5 bases and has significant differences in base types compared with those of Cas13T_9 and Cas13T_15).

As observed from the secondary structure of the DR sequence associated with Cas13Z_2, it includes a stem structure composed of 5'-GC-3' and its complementary bases, followed by a small bulge structure, then a stem structure composed of 5'-GU-3' and its complementary bases, immediately followed by a bulge structure composed of 5+5 bases, subsequently a stem structure composed of 5'-CCUU-3' and its complementary bases, and finally a terminal loop structure composed of 8 bases.

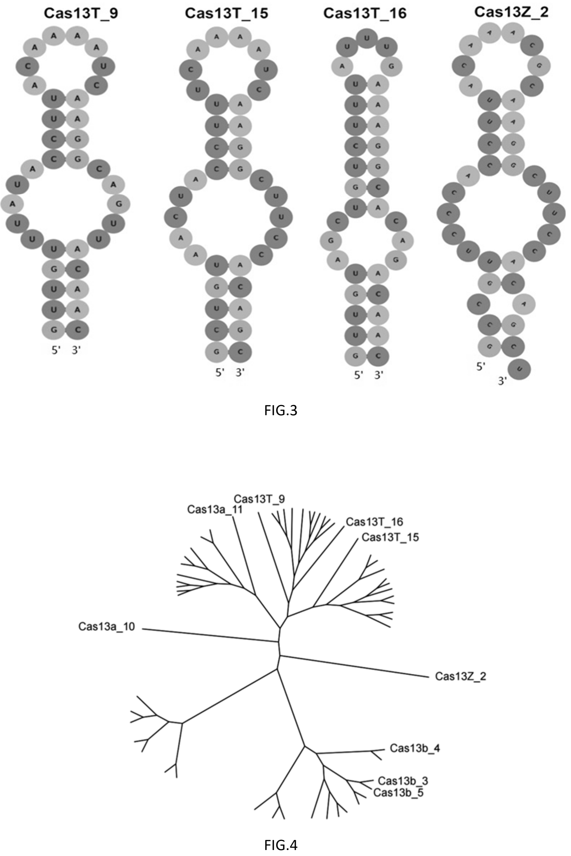

Multiple sequence alignment was performed between the aforementioned Cas13 proteins and previously identified Cas13 family proteins using sequence alignment software, and a phylogenetic tree was constructed. The construction result is shown in FIG. 4. As can be seen from FIG. 4, Cas13a_10 and Cas13a_11 are located on two relatively close branches of the phylogenetic tree; Cas13b_3, Cas13b_4, and Cas13b_5 are relatively close to each other on the phylogenetic tree, with Cas13b_3 and Cas13b_5 being even closer to each other. Cas13T_9, Cas13T_15, and Cas13T_16 have a close evolutionary relationship on the phylogenetic tree; and Cas13Z_2 has a relatively distant evolutionary relationship with all other Cas13 proteins on the phylogenetic tree.

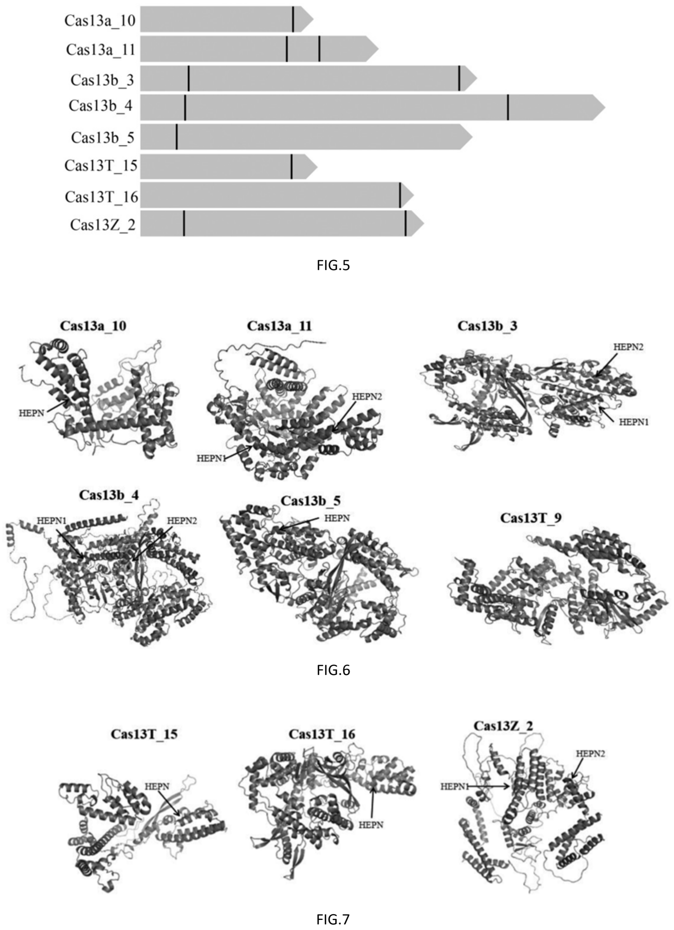

The inventors also performed functional annotation and multiple sequence alignment on each of the aforementioned Cas13 proteins using a publicly available Cas protein database, analyzed the sequence-conserved regions of these nine Cas13 proteins, and identified the positions of the conserved RXXXXH motifs in these nine Cas13 proteins; the identification results are shown in FIG. 5.

As can be seen from FIG. 5, Cas13a_10, Cas13b_5, Cas13T_15, and Cas13T_16 each contain only one RXXXXH motif-among them, the RXXXXH motifs of Cas13a_10, Cas13T_15, and Cas13T_16 are closer to the C-terminus, while the RXXXXH motif of Cas13b_5 is closer to the N-terminus. Cas13a_11, Cas13b_3, Cas13b_4, and Cas13Z_2 each contain two RXXXXH motifs: the two RXXXXH motifs of Cas13a_11 are closer to the C-terminus, and the two RXXXXH motifs of Cas13b_3, Cas13b_4, and Cas13Z_2 are closer to the two ends (N-terminus and C-terminus).

Specifically, the single RXXXXH motif of Cas13a_10 is located at positions 490 to 495; the two RXXXXH motifs of Cas13a_11 are located at positions 453 to 458 and 581 to 586, respectively; the two RXXXXH motifs of Cas13b_3 are located at positions 125 to 130 and 1079 to 1084, respectively; the two RXXXXH motifs of Cas13b_4 are located at positions 148 to 153 and 1176 to 1181, respectively. The single RXXXXH motif of Cas13b_5 is located at positions 128 to 133. The single RXXXXH motif of Cas13T_15 is located at positions 480 to 486. The single RXXXXH motif of Cas13T_16 is located at positions 800 to 805. The two RXXXXH motifs of Cas13Z_2 are located at positions 143 to 148 and 813 to 818, respectively. No obvious RXXXXH motif was found in the Cas13T_9 protein. The RXXXXH motif is a potential catalytic active site of Cas13 proteins; mutations in this domain may generate Cas13 protein variants with inactivated nuclease activity, and substantially retaining the ability to bind to guide RNA and target RNA.

The applicant also predicted the three-dimensional structures of the aforementioned Cas13 proteins using protein structure prediction software; details can be seen in FIGS. 6 to 7. Cas13b_3, Cas13b_4's two RXXXXH motifs are very close to the N-terminus and C-terminus in the protein sequence, but are very close to each other in the three-dimensional structure (see FIG. 6). The two RXXXXH motifs of Cas13Z_2 are relatively close to the N-terminus and C-terminus, but appear relatively close to each other in the three-dimensional structure (see FIG. 7).

### Example 2. Activity Verification of Cas13 Proteins

To verify whether the nine Cas13 proteins (Cas13a_10, Cas13a_11, Cas13b_3, Cas13b_4, Cas13b_5, Cas13T_9, Cas13T_15, Cas13T_16, and Cas13Z_2) have RNA nuclease activity, this example provides an experiment for verifying the cleavage activity of these nine Cas13 proteins. The specific experimental process is as follows:
(1) The codon-optimized polynucleotides (optimized for better expression in human cells) encoding Cas13a_10, Cas13a_11, Cas13b_3, Cas13b_4, Cas13b_5, Cas13T_9, Cas13T_15, Cas13T_16, and Cas13Z_2 (SEQ ID NOs. 10-18) were respectively cloned into the p23_puro_NPLS_Cas13_msfGFP_NLS_3xFlag_C plasmid carrying the gene encoding green fluorescent protein (GFP) (SEQ ID NO. 28). The nucleotide sequence of the p23_puro_NPLS_Cas13_msfGFP_NLS_3xFlag_C plasmid is shown in SEQ ID NO. 29, with restriction enzyme sites Nhel:Xbal. This yielded Cas13 protein recombinant plasmids. In the Cas13 protein recombinant plasmids, the polynucleotide of the Cas13 protein replaced the sequence at positions 3472 to 6369 in the p23_puro_NPLS_Cas13_msfGFP_NLS_3xFlag_C plasmid.
(2) The mCherry reporter gene recombinant plasmid was obtained; the nucleotide sequence of the mCherry reporter gene recombinant plasmid is shown in SEQ ID NO. 30.
(3) Spacer_1 to Spacer_10 (SEQ ID NOs. 33-42) are spacer sequences targeting different positions of mCherry (SEQ ID NO. 31), and NT-spacer (SEQ ID NO. 43) is a spacer sequence that does not target mCherry. For specific sequence design, see Table 3.

**Table 3 Spacer Sequences Targeting mCherry and Non-Targeting mCherry**

| Name | Spacer Sequence |
|---|---|
| spacer_1 | GCAGCTTCACCTTGTAGATGAACTCGCCGT(SEQ ID NO.33) |
| spacer_2 | GTTCATCACGCGCTCCCACTTGAAGCCCTC(SEQ ID NO.34) |
| spacer_3 | TGCTTCACGTAGGCCTTGGAGCCGTACATG(SEQ ID NO.35) |
| spacer_4 | TCAGCTTGGCGGTCTGGGTGCCCTCGTAGG(SEQ ID NO.36) |
| spacer_5 | TTGATGATGGCCATGTTATCCTCCTCGCCC(SEQ ID NO.37) |
| spacer_6 | CCCATGGTTTTCTTCTGCATTACGGGGCCG(SEQ ID NO.38) |
| spacer_7 | CAGGATGTCCCAGGCGAAGGGCAGGGGGCC(SEQ ID NO.39) |
| spacer_8 | TGTAGATGAACTCGCCGTCCTGCAGGGAGG(SEQ ID NO.40) |
| spacer_9 | TTCAGCCTCTGCTTGATCTCGCCCTTCAGG(SEQ ID NO.41) |
| spacer_10 | CCTCAGCGTCGTAGTGGCCGCCGTCCTTCA(SEQ ID NO.42) |
| NT-spacer | CGTCTGGCCTTCCTGTAGCCAGCTTTCATC(SEQ ID NO.43) |

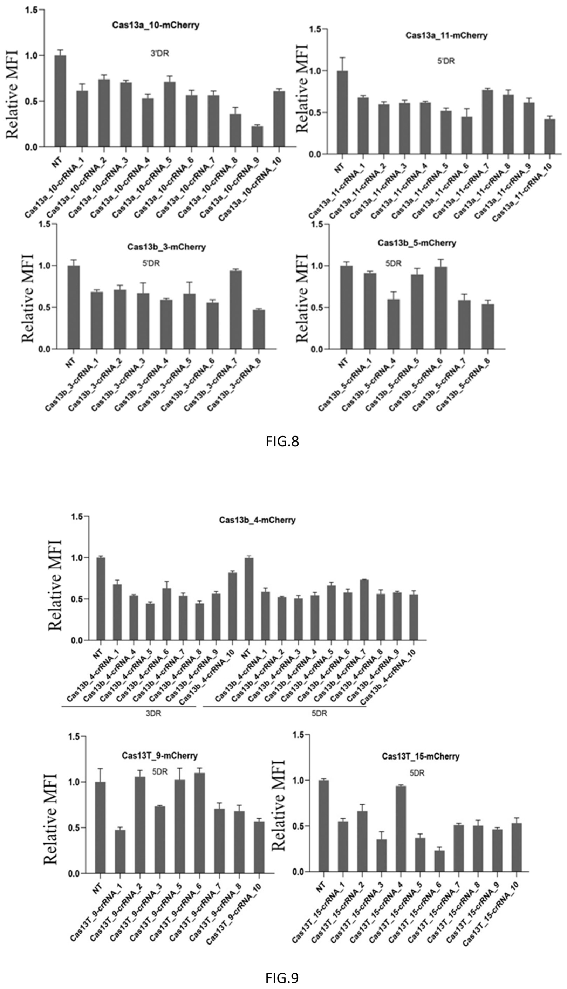

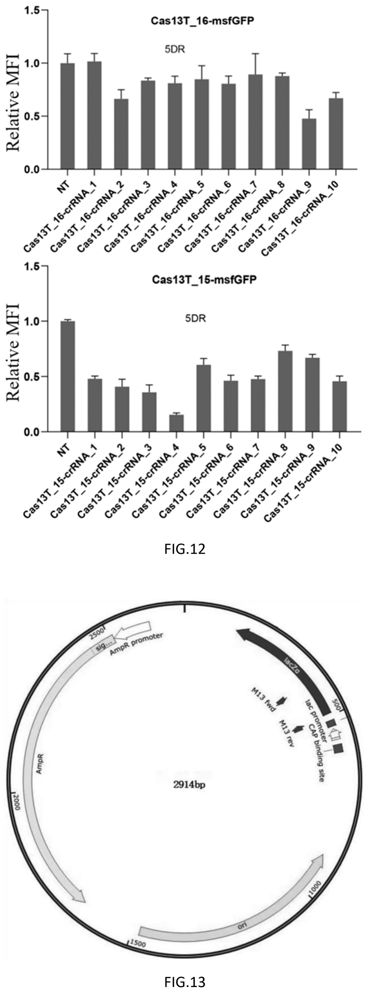

Theoretically, during the maturation of the guide RNA precursor in the CRISPR/Cas13 system, two types of guide RNA (i.e., crRNA) structures can be generated: direct repeat (DR) sequence + spacer sequence (i.e., 5'DR, where the DR sequence is linked to the 5' end of the spacer sequence) and spacer sequence + direct repeat (DR) sequence (i.e., 3'DR, where the DR sequence is linked to the 3' end of the spacer sequence). Since the DR sequences of Cas13a_10 and Cas13a_11 are identical, their DR sequences are named DR-Cas13a_10/11. For Cas13a_10, the inventors constructed a guide RNA with a 3'DR structure, which is named Cas13a_10-crRNA_1 in FIG. 8; for Cas13a_11, the inventors constructed a guide RNA with a 5'DR structure, which is named Cas13a_11-crRNA_1 in FIG. 8. Similarly, DR-Cas13b_3, DR-Cas13b_4, DR-Cas13b_5, DR-Cas13T_9, DR-Cas13T_15, DR-Cas13T_16, and DR-Cas13Z_2 were respectively linked to the 3' end or 5' end of spacer_1 to spacer_10 to construct guide RNAs targeting the reporter gene mCherry; additionally, the DR sequence of each aforementioned Cas protein was respectively linked to the 3' end or 5' end of NT-spacer to construct guide RNAs that do not target the reporter gene mCherry.
(4) The coding sequences of the guide RNAs targeting the reporter gene mCherry and the guide RNAs not targeting the reporter gene mCherry were respectively cloned into the U6-BbSl-PUC57 plasmid (purchased from Sangon Biotech (Shanghai) Co., Ltd.) after restriction enzyme digestion (restriction enzyme site: BbSI). The plasmid map of the U6-BbSI-PUC57 plasmid is shown in FIG. 13, and the plasmid nucleotide sequence is shown in SEQ ID NO. 32. This yielded guide RNA recombinant plasmids; among them, both the guide RNAs targeting mCherry and the guide RNAs not targeting mCherry are composed of direct repeat (DR) sequences and spacer sequences. The structures of the mCherry-targeting guide RNAs and non-mCherry-targeting guide RNAs used for the nine Cas13 proteins are shown in Table 4.

**Table 4 Guide RNAs**

| Cas13 Protein | Guide RNAs Targeting mCherry | Non-mCherry-Targeting Guide RNAs for Comparison (NT) |
|---|---|---|
| Cas13a_10 | 3'DR | 3'DR |
| Cas13a_11 | 5'DR | 5'DR |
| Cas13b_3 | 5'DR | 5'DR |
| Cas13b_4 | 3'DR, 5'DR | 3'DR, 5'DR |
| Cas13b_5 | 5'DR | 5'DR |
| Cas13T_9 | 5'DR | 5'DR |
| Cas13T_15 | 5'DR | 5'DR |
| Cas13T_16 | 5'DR | 5'DR |
| Cas13Z_2 | 5'DR | 5'DR |

(5) Human HEK293T cells (purchased from ATCC) were seeded in a 48-well plate at a density of 1×104 cells/well. Using LIPOFECTAMINE 3000 and P3000^{™} reagents (purchased from ThermoFisher, catalog number L3000015), the Cas13 protein recombinant plasmid, guide RNA recombinant plasmid (containing guide RNA targeting the mCherry reporter gene), and mCherry reporter gene recombinant plasmid were co-transfected into human HEK293T cells to obtain transfected human HEK293T cells of the experimental group. The Cas13 protein recombinant plasmid, guide RNA recombinant plasmid (containing guide RNA not targeting the mCherry reporter gene), and mCherry reporter gene recombinant plasmid were co-transfected into human HEK293T cells to obtain transfected human HEK293T cells of the negative control group. Human HEK293T cells without any plasmid transfection served as the blank control group. The transfected human HEK293T cells of the experimental group, transfected human HEK293T cells of the negative control group, and blank control group were cultured in a cell incubator at 37°C with 5% (v/v) CO2.
(6) After 48 hours of culture, microscopic observation revealed that the growth and morphology of transfected human HEK293T cells in the experimental group were similar to those in the negative control group and blank control group. The transfected human HEK293T cells were observed under a fluorescence microscope, and finally detected and quantitatively analyzed by flow cytometry; the analysis results are shown in FIGS. 8-10. Flow cytometry (FCM) analysis showed that in the negative control group, the guide RNA not targeting the mCherry reporter gene failed to knock down the expression of mCherry fluorescent signal. Compared with the negative control group, the mCherry fluorescent signal intensity of transfected human HEK293T cells in the experimental group was significantly reduced, indicating that all nine Cas13 proteins could effectively knock down the mRNA level of mCherry using the guide RNA targeting mCherry, thereby reducing the expression level of mCherry protein. When using the guide RNA with a 3'DR structure (Cas13a_10-crRNA9), Cas13a_10 showed a significant knockdown effect on mCherry expression, with the fluorescent signal intensity reduced by approximately 80% (when using crRNA_9). When using guide RNAs with specific structures, Cas13T_15, Cas13T_16, and Cas13Z_2 all exhibited good knockdown effects on mCherry expression, with a substantial reduction in fluorescent signal intensity. Cas13a_11, Cas13b_3, Cas13b_4, Cas13b_5, Cas13T_9, and Cas13T_16 also showed knockdown effects on mCherry expression, significantly reducing the fluorescent signal intensity. Comparison revealed that the degree of reduction in fluorescent signal intensity varied when using spacer sequences (crRNAs) targeting different positions of the mCherry plasmid, indicating that spacer sequences targeting different positions of the target RNA affect the editing efficiency of Cas13 proteins. For example, Cas13b_4 showed an obvious preference for guide RNAs with two structures (3'DR and 5'DR); among them, when Cas13b_4 was guided by crRNA with 3'DR, the knockdown effect of mCherry corresponding to some spacer sequences was relatively better.

2. The inventors also conducted comparative experiments between Cas13d and Cas13b_3, Cas13T_15, Cas13T_16, Cas13b_4, and Cas13T_9 using specific crRNAs, as follows:

### (1) Construction of Cas13d recombinant expression plasmid

The nucleotide sequence encoding Cas13d was optimized for mammalian codons, with the nucleotide sequence shown in SEQ ID NO. 56. Using the same method as in Example 2, Step 1(1), a Cas13d recombinant plasmid was constructed. In the Cas13d protein recombinant plasmid, the polynucleotide encoding the Cas13d protein replaced the sequence at positions 3472 to 6369 in the p23_puro_NPLS_Cas13_msfGFP_NLS_3xFlag_C plasmid.
(2) The mCherry reporter gene recombinant plasmid was obtained; the nucleotide sequence of the mCherry reporter gene recombinant plasmid is shown in SEQ ID NO. 30.
(3) Guide RNA recombinant plasmids for comparison were designed. Cas13d, which is known in the art to have Cas13 activity, was used as a comparative reference to verify the cleavage activity of some Cas13 proteins involved in the present disclosure. The DR sequence used for Cas13d is shown in SEQ ID NO. 57. Two sets of experiments were designed as needed, where:

In the first set of experiments, the spacer sequence used was spacer_1 (SEQ ID NO. 33). The guide RNA (crRNA) used for Cas13d was Cas13d-DR-spacer_1 (SEQ ID NO. 58), and the crRNAs used for Cas13T_9 and Cas13Z_2 were DR-Cas13T_9-spacer_1 (SEQ ID NO. 59) and DR-Cas13Z_2-spacer_1 (SEQ ID NO. 60), respectively.

In the second set of experiments, the spacer sequence used was spacer_6 (SEQ ID NO. 38). The crRNA used for Cas13d was Cas13d-DR-spacer_6 (SEQ ID NO. 61), and the crRNAs used for Cas13b_3, Cas13T_15, and Cas13T_16 were DR-Cas13b_3-spacer_6 (SEQ ID NO. 62), DR-Cas13T_15-spacer_6 (SEQ ID NO. 63), and DR-Cas13T_16-spacer_6 (SEQ ID NO. 19), respectively.

In addition, for each Cas13 protein in the first and second sets of experiments, a non-targeting control group was set up respectively. The spacer sequence used in all non-targeting control groups was NT-spacer (SEQ ID NO. 43), the DR sequence was the corresponding DR sequence of each Cas13 protein, and the crRNA sequence structure was DR-NT-spacer (5'DR).

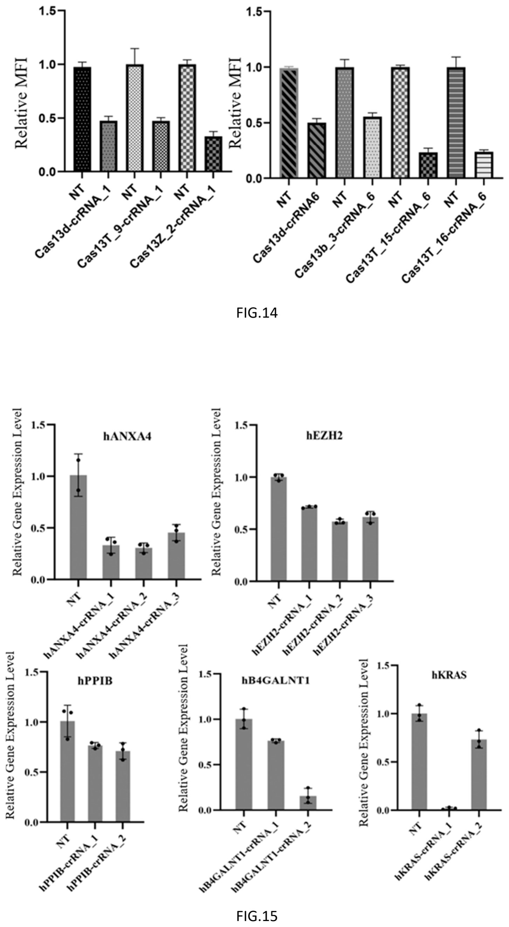

Using the method described in Example 2, Step 1, the Cas13 proteins and their corresponding crRNAs involved in each set of experiments were cloned and constructed into the p23_puro_NPLS_Cas13_msfGFP_NLS_3xFlag_C plasmid and U6-BbSI-PUC57 plasmid, respectively. Subsequently, they were co-transfected with the mCherry reporter gene recombinant plasmid into human HEK293T cells (purchased from ATCC) for cleavage activity detection using the same method. Flow cytometry detection and analysis showed that at one site (the target site of spacer_1), the cleavage activity of Cas13Z_2 was higher than that of Cas13d, and the cleavage activity of Cas13T_9 was comparable to that of Cas13d; at the other site (the target site of spacer_6), the cleavage efficiency of Cas13T_15 and Cas13T_16 was higher than that of Cas13d, and the cleavage activity of Cas13b_3 was slightly lower than that of Cas13d (see FIG. 14).

### Example 3 Verification of Collateral Activity (i.e., Non-Specific Cleavage Activity) of Cas13 Proteins

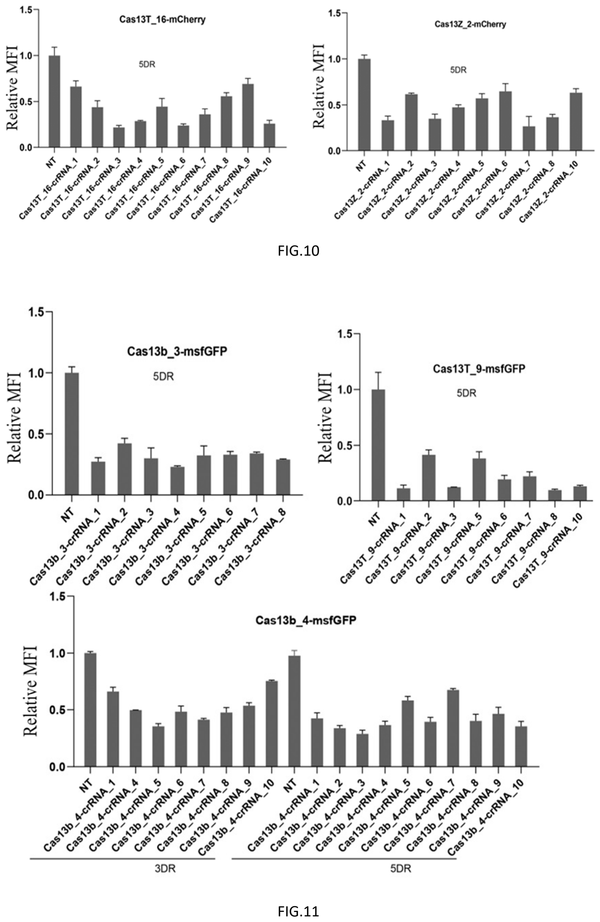

To verify the collateral activity of the aforementioned Cas13 proteins, the applicant also conducted verification experiments on the collateral activity of some Cas13 proteins. The experimental process is as follows:
(1) The same mCherry-targeting guide RNAs as those used in Example 2 were adopted, and the experiment of Example 2 was repeated. Specifically, the Cas13 protein recombinant plasmids carrying the nucleotide sequences encoding Cas13b_3, Cas13b_4, Cas13T_9, Cas13T_15, and Cas13T_16 were respectively co-transfected into human HEK293T cells together with the mCherry reporter gene recombinant plasmid and the guide RNA recombinant plasmid (containing the guide RNA that does not target the mCherry reporter gene).
(2) Flow cytometry was used to analyze the knockdown efficiency of the GFP reporter gene in the cells 48 hours after transfection. The knockdown efficiency of GFP represents the non-specific cleavage activity of Cas13, and the experimental results are shown in FIGS. 11 to 12.

As can be seen from FIGS. 11 to 12, Cas13b_3, Cas13b_4, Cas13T_9, Cas13T_15, and Cas13T_16 all exhibit non-specific cleavage activity, and the guide RNAs corresponding to different spacer sequences also have an impact on the level of collateral activity. For example, in FIG. 11, when crRNA_1, crRNA_3, crRNA_8, and crRNA_10 in the guide RNAs were used, Cas13T_9 showed relatively higher non-specific cleavage activity compared with other guide RNAs; for Cas13b_4, there were also differences in the level of collateral activity when crRNAs with 3'DR and 5'DR structures were adopted. As shown in FIG. 12, when crRNA_4 was used, Cas13T_15 exhibited higher collateral activity. The collateral activity of the aforementioned Cas13 proteins can be used for the detection of DNA and/or RNA molecules.

### Example 4 Verification of Cleavage Activity on Endogenous Genes in Eukaryotic Cells

To verify the cleavage activity of the Cas13 proteins of the present disclosure on endogenous genes in eukaryotic cells, the applicant selected multiple endogenous genes of eukaryotic cells-ANXA4, B4GALNT1, EZH2, PPIB, and KRAS-as target genes, and determined the cleavage activity using the qPCR relative quantification method.

The applicant selected Cas13T_15 as the verification target and designed spacer sequences targeting the mRNAs of the endogenous genes ANXA4, B4GALNT1, EZH2, PPlB, and KRAS, as shown in Table 5 below:

**Table 5 Spacer Sequences Targeting mRNAs of Human Endogenous Genes ANXA4, B4GALNT1, EZH2, PPIB, and KRAS**

| Spacer Sequence Name | Sequence |
|---|---|
| hANXA4_spacer1 | TTAGGCAGCCCTCATCAGTGCCGGCTCCCT(SEQ ID NO.44) |
| hANXA4_spacer2 | GGCCAGGATCTCAATTAGGCAGCCCTCATC(SEQ ID NO.45) |
| hANXA4_spacer3 | ATGGCCCTTCGCAGCTCTTGCACGTCATAC(SEQ ID NO.46) |
| hB4GALNT1_spacer1 | CCTCCTGACCAGAAGCTGCCTGAAGGCTCA(SEQ ID NO.47) |
| hB4GALNT1_spacer2 | CTGGTATACCTCCTGACCAGAAGCTGCCTG(SEQ ID NO.48) |
| hEZH2_spacer1 | CAAATGCTGGTAACACTGTGGTCCACAAGG(SEQ ID NO.49) |
| hEZH2_spacer2 | GAGAGCAGCAGCAAACTCCTTTGCTCCCTC(SEQ ID NO.50) |
| hEZH2_spacer3 | TTTGCTCCCTCCAAATGCTGGTAACACTGT(SEQ ID NO.51) |
| hPPlB_spacer1 | GGCCCGTAGTGCTTCAGTTTGAAGTTCTCA(SEQ ID NO.52) |
| hPPIB_spacer2 | GCTTCAGTTTGAAGTTCTCATCGGGGAAGC(SEQ ID NO.53) |
| hKRAS_spacer1 | CCTACTAGGACCATAGGTACATCTTCAGAG(SEQ ID NO.54) |
| hKRAS_spacer2 | GTGTCTACTGTTCtAGAAGGCAAATCACAT(SEQ ID NO.55) |

DR-Cas13T_15 was respectively linked to the 5' end of each spacer sequence in Table 5 (i.e., using guide RNAs in the 5'DR form) to obtain guide RNAs targeting the mRNAs of each endogenous gene, and an NT guide RNA that does not target any endogenous gene RNA was set as a control. The coding sequences of each guide RNA were cloned into the U6-BbSI-PUC57 plasmid using the same method as in Example 2 to obtain guide RNA recombinant plasmids targeting the mRNAs of different endogenous genes.

The Cas13T_15 recombinant plasmid obtained in Example 1, the aforementioned guide RNA recombinant plasmids targeting the mRNAs of different endogenous genes, and the NT control guide RNA were respectively transfected (in the same transfection manner as in Example 2) into HEK293 cells (purchased from ATCC) to obtain the experimental group and control group. Then, RT-PCR was used to determine the expression of endogenous gene mRNAs to evaluate the knockdown efficiency, compared with control cells transfected with the Cas13T_15 recombinant plasmid/NT control guide RNA recombinant plasmid. After culturing in a cell incubator at 37°C with 5% (v/v) CO₂ for 48 hours, the cells were harvested, RNA was extracted and reverse-transcribed into cDNA, and qPCR relative quantitative analysis was performed using GAPDH as the internal reference gene to compare the reduction in mRNA expression levels of each endogenous gene relative to the control group.

The quantitative analysis results are shown in FIG. 15, which indicate that Cas13T_15 knocks down the mRNA expression of each endogenous gene to varying extents. When specific guide RNAs were used, the knockdown on ANXA4 mRNA, KRAS mRNA, and B4GALNT1 mRNA expression have efficiency exceeding 60%, while the mRNA expression of EZH2 and PPIB also showed significant mRNA knockdown. This also confirms that Cas13T_15 can exert targeted cleavage effects in eukaryotic cells.

Obviously, the above examples are merely illustrative for clarity and are not intended to limit the implementation modes. For those of ordinary skill in the art, other different forms of changes or modifications can be made on the basis of the above description. It is unnecessary and impossible to enumerate all implementation modes here. However, the obvious changes or modifications derived therefrom still fall within the protection scope of the present invention.

## Claims

1. A Cas13 protein, wherein the Cas13 protein is a protein as defined in (a) or (b) below:
(a) a protein comprising an amino acid sequence represented by SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, or SEQ ID NO. 9;
(b) a protein being derived by substituting, deleting, or adding one or more amino acids in the amino acid sequence defined in (a), and substantially retaining the biological function of the sequence from which it is derived.

2. The Cas13 protein according to claim 1, wherein the protein in (b) has an amino acid sequence having at least 85% sequence identity to that in (a), and substantially retains the biological function of the sequence from which it is derived.

3. The Cas13 protein according to claim 2, wherein the protein in (b) has an amino acid sequence having at least 95% sequence identity to that in (a), and substantially retains the biological function of the sequence from which it is derived.

4. A fusion protein comprising the Cas13 protein according to any one of claims 1-3 and a heterologous functional domain.

5. The fusion protein according to claim 4, wherein the heterologous functional domain comprises at least one selected from the group consisting of a deaminase, a reporter protein, a detection label, a localization signal, a protein targeting domain, a DNA-binding domain, an epitope tag, a transcriptional activation functional domain, a transcriptional repression functional domain, a nuclease, a methylation-active enzyme, a demethylase, a transcript release factor, an RNA-cleaving polypeptide, a nucleic acid ligase, a histone modification activator, and a nucleic acid-binding activator.

6. The fusion protein according to claim 5, wherein the heterologous functional domain is fused to the N-terminus or C-terminus of the Cas13 protein, or the heterologous functional domain is fused internally within the Cas13 protein.

7. The fusion protein according to claim 5, wherein the localization signal is a nuclear localization signal or a nuclear export signal.

8. A CRISPR-Cas system, wherein the CRISPR-Cas system comprises the Cas13 protein according to any one of claims 1-3 and a guide RNA, or the CRISPR-Cas system comprises the fusion protein according to any one of claims 4-7 and a guide RNA; wherein the guide RNA is capable of binding to the Cas13 protein to form a complex and guiding the complex to contact a target RNA.

9. The CRISPR-Cas system according to claim 8, wherein the guide RNA comprises a spacer sequence and a direct repeat sequence; the spacer sequence has a length of 15-60 nucleotides; and the direct repeat sequence has a length of 15-40 nucleotides.

10. The CRISPR-Cas system according to claim 9, wherein there is complementarity of at least 15 nucleotides between the spacer sequence and the target RNA.

11. A polynucleotide encoding the Cas13 protein according to any one of claims 1-3, or encoding the fusion protein according to any one of claims 4-7, or encoding the CRISPR-Cas system according to any one of claims 8-10.

12. A vector carrying the polynucleotide according to claim 11.

13. The vector according to claim 12, wherein the polynucleotide carried by the vector is linked to regulatory elements.

14. The vector according to claim 13, wherein the regulatory elements comprise at least one selected from the group consisting of a promoter, an enhancer, a polyadenylation signal, a terminator, a protein degradation signal, a transposon, a leader sequence, a polyadenylic acid sequence, and a marker gene.

15. The vector according to any one of claims 12-14, wherein the vector comprises at least one selected from the group consisting of a viral vector and a non-viral vector.

16. The vector according to claim 15, wherein the viral vector comprises at least one selected from the group consisting of a retroviral vector, a phage vector, an adenoviral vector, an adeno-associated viral vector, a vaccinia viral vector, a hybrid viral vector, a baculoviral vector, a herpes simplex viral vector, and a lentiviral vector.

17. The vector according to claim 15, wherein the non-viral vector comprises a plasmid vector.

18. A delivery system carrying the Cas13 protein according to any one of claims 1-3, the fusion protein according to any one of claims 4-7, the CRISPR-Cas system according to any one of claims 8-10, the polynucleotide according to claim 11, or the vector according to any one of claims 12-17.

19. The delivery system according to claim 18, wherein the delivery carrier of the delivery system comprises at least one selected from the group consisting of a liposomal carrier, a chitosan carrier, a polymer carrier, a nanoparticle carrier, an exosome carrier, an ectosome carrier, a microbubble carrier, a viral vector, a gene gun, and an electroporation device.

20. A host cell carrying the Cas13 protein according to any one of claims 1-3, the fusion protein according to any one of claims 4-7, the CRISPR-Cas system according to any one of claims 8-10, the polynucleotide according to claim 11, or the vector according to any one of claims 12-17.

21. A model, wherein the model is a plant model or an animal model; and the plant model or animal model comprises the host cell according to claim 20.

22. A method for gene editing, comprising contacting a target RNA with the Cas13 protein according to any one of claims 1-3, the fusion protein according to any one of claims 4-7, the CRISPR-Cas system according to any one of claims 8-10, the polynucleotide according to claim 11, the vector according to any one of claims 12-17, or the host cell according to claim 20.

23. The method according to claim 22, wherein the guide RNA binds to the Cas13 protein to form a complex, and guides the complex to contact the target RNA, so as to perform gene editing on the target RNA.

24. A gene-edited product obtained by performing gene editing on a target RNA using the method according to claim 22 or 23.

25. Use of the Cas13 protein according to any one of claims 1-3, the fusion protein according to any one of claims 4-7, the CRISPR-Cas system according to any one of claims 8-10, the polynucleotide according to claim 11, the vector according to any one of claims 12-17, the delivery system according to claim 18 or 19, the host cell according to claim 20, or the method according to any one of claims 22-23 in gene editing.

26. A kit, comprising the Cas13 protein according to any one of claims 1-3, the fusion protein according to any one of claims 4-7, the CRISPR-Cas system according to any one of claims 8-10, the polynucleotide according to claim 11, the vector according to any one of claims 12-17, the delivery system according to claim 18 or 19, or the host cell according to claim 20.

27. A nucleic acid detecting method, wherein the method comprises detecting a nucleic acid in a test sample using the kit according to claim 26.

28. Use of the Cas13 protein according to any one of claims 1-3, the fusion protein according to any one of claims 4-7, the CRISPR-Cas system according to any one of claims 8-10, the polynucleotide according to claim 11, the vector according to any one of claims 12-17, the delivery system according to claim 18 or 19, the host cell according to claim 20, the kit according to claim 26, or the method according to claim 27 in nucleic acid detection.

29. Use of the Cas13 protein according to any one of claims 1-3, the fusion protein according to any one of claims 4-7, the CRISPR-Cas system according to any one of claims 8-10, the polynucleotide according to claim 11, the vector according to any one of claims 12-17, the delivery system according to claim 18 or 19, or the host cell according to claim 20 in the manufacture of a medicament for treating a disorder or disease in a subject in need thereof.

30. A method for treating a disorder or disease in a subject in need thereof, the method comprising:
administering to the subject a composition comprising the CRISPR-Cas system according to any one of claims 8-10 or a polynucleotide encoding the system; wherein there is complementarity of at least 15 nucleotides between the spacer sequence and a target RNA associated with the disease or disorder; wherein the Cas13 protein binds to the guide RNA to form a complex, the complex binds to the target RNA, and the Cas13 protein edits the target RNA, thereby achieving the purpose of treating the disorder or disease in the subject;
preferably, the disorder or disease is cancer, an infectious disease, or another disease;
preferably, the cancer is Wilms' tumor, Ewing sarcoma, a neuroendocrine tumor, a glioblastoma, a neuroblastoma, a melanoma, skin cancer, breast cancer, colon cancer, rectal cancer, prostate cancer, liver cancer, renal cancer, pancreatic cancer, lung cancer, biliary cancer, cervical cancer, endometrial cancer, esophageal cancer, gastric cancer, head and neck cancer, medullary thyroid carcinoma, ovarian cancer, glioma, lymphoma, leukemia, myeloma, Hodgkin lymphoma, non-Hodgkin lymphoma, or bladder cancer;
preferably, the leukemia comprises acute lymphoblastic leukemia (ALL), acute myeloid leukemia (AML), chronic lymphocytic leukemia (CLL), or chronic myeloid leukemia (CML);
preferably, the another disease comprises a neurological disease, an otological disease, a muscle abnormality, or an ophthalmological disease;
preferably, the neurological disease comprises a neurodevelopmental abnormality, Angelman syndrome, or amyotrophic lateral sclerosis (ALS);
preferably, the otological disease comprises dominantly inherited deafness;
preferably, the muscle abnormality comprises spinal muscular atrophy (SMA);
preferably, the ophthalmological disease comprises Leber congenital amaurosis type 2 (LCA2).
